# EUROPÄISCHE PATENTSCHRIFT

(11) **EP 1 102 988 B1**
(45) Veröffentlichungstag und Bekanntmachung des Hinweises auf die Patenterteilung: **26.10.2005**
(21) Anmeldenummer: 99940137.5
(22) Anmeldetag: 04.08.1999
(51) Int. Cl.: G01N 33/50, G01N 33/49

(54) **VERFAHREN ZUR BESTIMMUNG DER IMMUNABWEHRAKTIVITÄT DES BLUTES SOWIE TESTKIT HIERFÜR**
METHOD OF DETERMINING THE IMMUNE RESPONSE ACTIVITY OF BLOOD AND TEST KIT THEREFOR
PROCEDE D'EVALUATION DE L'ACTIVITE DE LA REPONSE IMMUNITAIRE DU SANG ET TROUSSE D'ESSAI CORRESPONDANTE

(30) Priorität: 07.08.1998 DE 19835721
(43) Veröffentlichungstag der Anmeldung: 30.05.2001
(73) Patentinhaber: EDI (Experimentelle & Diagnostische Immunologie) Gmb, 72770 Reutlingen (DE)
(72) Erfinder: SCHMOLZ, Manfred, D-72810 Gomaringen (DE)
(74) Vertreter: Patentanwälte Ruff, Wilhelm, Beier, Dauster & Partner
(86) Internationale Anmeldenummer: PCT/EP1999/005635
(87) Internationale Veröffentlichungsnummer: WO 2000/008464

(56) Entgegenhaltungen:
- DE-A- 2 415 704
- DE-A- 4 128 923
- US-A- 5 128 270

## Beschreibung

Die Erfindung betrifft ein Verfahren zur Bestimmung der Immunabwehraktivität des Blutes und einen Testkit zur Durchführung der Bestimmung.

Die Bestimmung der Immunabwehraktivität des Blutes wird vorgenommen, um festzustellen, ob blutführende Lebewesen, insbesondere Säugetiere und Menschen, eine Störung, insbesondere Schwäche des Immunsystems besitzen. Bei den bisherigen Methoden werden Blutproben nach der Entnahme aufgetrennt, wonach eine getrennte Behandlung und Untersuchung von weißen Blutkörperchen vorgenommen wird. Dabei wurde festgestellt, daß durch Lagerung und Transport, welche bis zu 24 Stunden dauern können und insbesondere im Sommer mit einer unerwünschten Wärmeeinwirkung verbunden sind, ein großer Teil der Aktivität der weißen Blutkörperchen verlorengehen kann. Untersuchungen ergaben, daß bereits innerhalb von einer Stunde manche Zellen 50 % ihrer Aktivität verlieren.

Die Trennung von weißen und roten Blutzellen wird vorgenommen, da die roten Blutzellen als Störfaktoren für die Aktivitätsbestimmung der weißen Blutzellen verantwortlich gemacht werden. Nach der Trennung werden die weißen Blutzellen in Puffermedium in Gegenwart von Nährsalzen und Vitaminen aufbewahrt. Es wurde gefunden, daß auch in einer solchen Nährlösung eine Veränderung der Aktivität möglich ist.

Gemäß einem anderen Ansatz wird das Vollblut in Gegenwart von Blutgerinnungsinhibitoren und Stimulantien inkubiert. Dies wird beispielsweise in der US 5128270 beschrieben. Hierin erfolgt das Inkubieren des Blutes unmittelbar auf die Blutentnahme bei ca. 37°C für 0,5 bis 22 Stunden, die Inkubierung des Vollblutes erfolgt allerdings nicht in Gegenwart von Nährmedium.

In der DE 2415704 wird eine Filtervorrichtung beschrieben, welche zum Abtrennen des beim Gerinnen und Zentrifugieren einer Blutprobe entstehenden Blutkuchens vom überstehenden Serum vorgesehen ist. Mit dieser Filtervorrichtung selbst kann das Blut entnommen werden. Die Vorrichtung besteht aus einem Spritzenzylinder mit einem passenden Ventilkolben zur Abtrennung der abgesetzten Blutkörperchen vom Überstand.

Der Erfindung liegt die Aufgabe zugrunde, die Zuverlässigkeit und Handhabung von Verfahren zur Bestimmung der Immunabwehraktivität zu verbessern.

Manche Blutzellen sind sofort zum Test geeignet. Die meisten Zellen müssen aber so aktiviert werden, daß ihr Stoffwechsel umgestellt wird, d.h. neue Gene aktiviert werden. Dies erfordert in der Regel mindestens 6 bis 8 Stunden. Die Zuverlässigkeit und Reproduzierbarkeit der Aktivitätsbestimmung können wesentlich verbessert werden, wenn eine Inkubierung und insbesondere Stimulierung der Blutzellen im wesentlichen unmittelbar nach der Blutentnahme vorgenommen wird, ohne daß eine Auftrennung in weiße und rote Blutzellen erfolgt. Dabei werden die weißen Blutkörperchen inkubiert und stimuliert, solange sie sich im Vollblut befinden. Während der Inkubation trennt sich das Blut von selbst auf, ohne daß eine Zentrifugation erforderlich ist. Im Überstand finden sich dann von den weißen Blutzellen abgegebene Botenstoffe (Cytokine), die ein Maß für die Aktivität der Zellen sind und beispielsweise durch ELISA gefunden und bestimmt werden können. Der Ausdruck Cytokine umfaßt neben den Cytokinen im engeren Sinne auch andere Botenstoffe, wie beispielsweise Histamin, Prostaglandine, Leukotriene usw..

Erfindungsgemäß sind somit ein Verfahren gemäß Anspruch 1 und ein Testkit gemäß Anspruch 12 vorgesehen.

Durch die Erfindung können in mehrfacher Hinsicht Fehler vermieden werden.
1. Systematische Fehler.
   Zellen reagieren normalerweise auf Stimulantien, zum Beispiel Medikamente, nicht allein allergisch, sondern auf den Verbund der Stimulantien mit Proteinen (Trägerproteinen), zum Beispiel Albumin oder Lipoprotein, oder im Verbund mit anderen Zellen, zum Beispiel Antigenpräsentierende Zellen (APC). Diese besitzen ihrerseits von Rezeptoren erkennbare Oberflächen. Die Anwesenheit von solchen costimulierenden Signalen ist zusätzlich erforderlich, um eine möglichst präzise Abbildung der natürlichen, auch in vivo zu erwartenden Aktivitäten der zu untersuchenden Zellen zu erreichen. Im Gegensatz zu Kulturen mit isolierten Leukozyten enthalten Vollblutkulturen diese Bestandteile und Signale, so daß die Reaktion der Blutzellen, solange sie sich im Vollblut befinden, den natürlichen Verhältnissen eher entspricht als in isoliertem Zustand. Es wurde auch gefunden, daß isolierte Leukozyten eine viel höhere Reaktion auf Stimulantien und Medikamente zeigen, als wenn sie im Vollblut enthalten sind, da im Vollblut die Stimulantien und Medikamente häufig an Erythrozyten und Trägerproteinen gebunden sind. Durch diese Bindung wird die Konzentration der Stimulantien und Medikamente abgepuffert, was zu einer allmählicheren Freisetzung der von den Zellen abgegebenen Botenstoffen führt.
2. Beseitigung von Handhabungsfehlern.
   Wie oben dargelegt, wurde gefunden, daß die roten Blutkörperchen die Aktivierung der weißen Blutzellen (Leukozyten) entgegen den ursprünglichen Vorstellungen nicht stören, sondern eher die Verhältnisse widerspiegeln, wie sie in vivo vorliegen. Durch die Abtrennung von Überstand nach der Inkubation kann jedoch in einfacher Weise eine nachträgliche Beeinflussung des Überstandes durch die roten Blutkörperchen (Erythrozyten) vermieden werden. Dies wirkt sich auch auf eine nach der Inkubation normalerweise erforderlich werdende Lagerung bzw. einen Versand günstig aus, indem durch das Einführen des Trennstempels die Sezernierung von Cytokinen in den Überstand augenblicklich unterbunden wird und dadurch die zellulären Reaktionen zeitlich sehr definiert beendet werden können.

Durch die Erfindung lassen sich auch pharmakologische und toxikologische Wirkungen auf das Immunsystem feststellen.

Erfindungsgemäß wird das Vollblut ohne vorherige Auftrennung extrakorporal mit einem Blutgerinnungsmittel und mindestens einem Stimulans (Aktivierungsmittel) zur Stimulierung von Blutzellen vermischt, das gesamte Blut inkubiert, um die Blutzellen zu stimulieren, eine Abtrennung von gebildetem Überstand vom Sediment vorgenommen und die Immunabwehraktivität aus dem Überstand bestimmt. Unter Blutgerinnungsmittel ist insbesondere ein Blutgerinnungsinhibitor zu verstehen, mit dessen Hilfe das Blut während und nach der Blutentnahme an der Gerinnung gehindert wird. Das Inkubieren erfolgt in Gegenwart eines Nährmediums. Vorzugsweise wird das Nährmedium mit dem Blutgerinnungsmittel, insbesondere Blutgerinnungsinhibitor, und insbesondere mit dem mindestens einen Stimulans zusammengebracht, bevor diese mit dem Blut vermischt werden. In der Regel wird das Nährmedium mit dem Blut im Volumenverhältnis 5:1 bis 1:5, insbesondere 1:1 bis 1:2, vermischt. Das Nährmedium kann in dem Gefäß vorgelegt werden, in dem die Inkubierung bzw. Kultivierung durchgeführt wird. Für reproduzierbare Ergebnisse ist es wichtig, daß die Inkubierung bei konstanter Temperatur von ca. 37 °C, vorgenommen wird. Dies kann in geeigneter Weise in einem Thermoblock geschehen, der vorzugsweise mehrere Aufnahmeöffnungen für Inkubationsgefäße besitzt. Neben der Verwendung von Thermoblöcken werden auch andere thermostatisch regulierbare Vorrichtungen von der Erfindung umfaßt. So kann die Inkubierung auch in einem Wasserbad oder beispielsweise in einem entsprechend temperierbaren Schrank o.ä. erfolgen. Die Inkubationsdauer liegt zwischen 8 und 60 Stunden, Als Inkubationsdauer können zweckmäßigerweise 24 oder 48 Stunden gewählt werden. Auch diese Festlegung begünstigt den Erhalt von reproduzierbaren Werten. Während der Inkubationsdauer setzen sich die roten und weißen Blutkörperchen in der Regel von selbst ab. Die Trennung des Überstandes von den Blutkörperchen wird deshalb ohne vorherige Zentrifugation vorgenommen. Die Aktivitätsbestimmung kann dann am Überstand durch Bestimmung der von den Blutzellen abgeschiedenen Botenstoffe (Cytokine) vorgenommen werden. Die Durchführung der Bestimmung erfolgt insbesondere in an sich bekannter Weise durch ELISA oder andere geeignete Testverfahren.

Das Vollblut wird in Gegenwart von mindestens einem Stimulans inkubiert.

In der Regel erfolgt die Aktivitätsbestimmung an einem anderen Ort als die Inkubation, oder die Aktivitätsbestimmung erfolgt aus anderen Gründen nicht unmittelbar nach Beendigung der Inkubation. Deshalb ist es erfindungsgemäß mit Vorteil vorgesehen, den Überstand bis zur Aktivitätsbestimmung gekühlt aufzubewahren, insbesondere bei Temperaturen von 0 bis 8 °C, insbesondere bei ca. 4 °C. In besonderen Fällen ist auch ein Eingefrieren möglich. Da der Überstand und vorzugsweise auch die abgesetzten Blutkörperchen nach der Trennung im Inkubationsgefäß verbleiben, wird das Sediment mit den Blutkörperchen mitgekühlt. Erfindungsgemäß wird das zur Blutentnahme verwendete Blutentnahmesystem auch als Inkubationsgefäß verwendet. Als Blutentnahmesystem wird vorzugsweise ein herkömmlicher Spritzenzylinder eingesetzt. Von der Erfindung werden jedoch auch weitere Blutentnahmesysteme umfaßt, wie beispielsweise evakuierte Glasröhrchen mit Gummidichtung, auf welche Kanülen aufsetzbar sind. Auch die Trennung des Überstandes von den Blutkörperchen wird im Blutentnahmesystem mit Hilfe einer zeitweise durchlässigen Trennwand vorgenommen. Besonders vorteilhaft ist hierzu ein im Blutentnahmesystem verschiebbarer Ventilkolben. Das zur Blutentnahme verwendete Blutentnahmesystem kann weiterhin mit Vorteil nach der Trennung von Überstand und Blutkörperchen auch als Kühl-, Transport- und insbesondere Versandbehälter verwendet werden. Der Versand kann in sogenannten Kühlblöcken erfolgen, die mit einem Kältespeichermedium gefüllt sind und vorzugsweise ein als Einstecköffnung dienendes Sackloch zur Aufnahme des Blutentnahmesystems, insbesondere des Spritzenzylinders, besitzen.

Als Spritzenzylinder für die Durchführung des erfindungsgemäßen Verfahrens eignen sich besonders im Handel unter der Bezeichnung "Monovette" (eingetragene Marke der Firma Sarstedt) befindliche Spritzenzylinder mit abbrechbarer Kolbenstange und einer den gesamten Querschnitt des Spritzenzylinders freigebenden Verschlußkappe auf der Seite der Nadel. Solche Spritzenzylinder können nach dem Abbrechen der Kolbenstange wie Reagenzgläser oder Zentrifugengläser gehandhabt werden. Nach der Inkubationszeit und dem Absetzen der Blutkörperchen kann ein dem Spritzenzylinder zugeordneter Ventilkolben bei abgenommener Kappe von oben eingeführt werden und in den Überstand hineingedrückt werden, wobei der Überstand durch den Ventilkolben hindurchfließt und bei Beendigung des Eindrückvorganges und Herausziehen eines Eindrückstabes von selbst schließt, so daß danach eine Vermischung zwischen den Blutkörperchen und dem Überstand nicht mehr möglich ist. Die im unteren Teil des Spritzenzylinders eingefangenen Blutkörperchen und der davon abgetrennte im oberen Teil befindliche Überstand werden bei der weiteren Handhabung im Spritzenzylinder belassen, bis eine Entnahme von Überstand zur Durchführung der Aktivitätsbestimmung erfolgt. Es sind bereits Spritzenzylinder im Handel, die zur Blutentnahme einen Blutgerinnungsinhibitor, zum Beispiel Heparin, insbesondere in lyophilisierter Form enthalten. Solche Spritzenzylinder enthalten meistens auch noch Kunststoffperlen, um eine schnelle Abtrennung der roten Blutkörperchen vom Überstand zu bewirken. Beim erfindungsgemäßen Verfahren ist vorzugsweise ebenfalls ein Blutgerinnungsinhibitor im Spritzenzylinder enthalten, nicht aber die normalerweise üblichen Kunststoffperlen. Mit Vorteil enthalten die Spritzenzylinder beim erfindungsgemäßen Verfahren auch schon den mindestens einen Stimulator, vorzugsweise ebenfalls in trockener, insbesondere lyophilisierter Form. Es ist aber auch möglich, den mindestens einen Stimulator getrennt aufzubewahren, zum Beispiel in Form einer stabilisierten Lösung, und ihn gesondert in dem Spritzenzylinder aufzunehmen, insbesondere kurz vor der Blutentnahme. Vor der Blutentnahme kann das gewünschte Volumen an Nährmedium in den Spritzenzylinder eingegeben werden, insbesondere durch Aufsaugen aus einer Einstechflasche. Bei der Blutentnahme erfolgt dann sofort eine Vermischung mit dem Nährmedium, in dem auch die vorgelegten Substanzen gelöst sind.

In einer weiteren bevorzugten Ausführungsform der Erfindung enthält das Blutentnahmesystem zum Zeitpunkt der Blutentnahme ausschließlich das Blutgerinnungsmittel, insbesondere den Blutgerinnungsinhibitor. Bei dieser Ausführungsform wird die Gefahr vermieden, daß durch eine versehentliche Reininjektion der vorgelegte Inhalt des Blutentnahmesystems (z.B. stimulantien und/oder Nährmedium) in den Menschen gelangen und hier u.U. schädigend wirken kann. Nach der Blutentnahme werden bei dieser Ausführungsform die weiteren Zusätze, wie beispielsweise Stimulantien und/oder Nährmedium, dem Blut in dem Blutentnahmesystem zugesetzt. Dies kann insbesondere durch Aufsaugen aus entsprechenden Einstechflaschen erfolgen.

Stimulantien können für sich allein oder in Kombination zu mehreren verwendet werden. Als Stimulantien kommen vor allem in Frage: Mitogene, das sind die Zellteilung aktivierende Stoffe. Hier gibt es zahlreiche verschiedene Arten. Diese dienen u.a. zur Aktivierung der verschiedenen Subtypen von Lymphozyten. Darüber hinaus gibt es auch partikuläre Aktivatoren von Granulozyten und Monozyten, wie etwa Zymosan und Bakterien. Granulozyten und Monozyten lassen sich auch durch lösliche signalstoffe wie LPS und Muramylpeptide aktivieren. Des weiteren können Antigene und Allergene sowie Antikörper gegen Oberflächen-Strukturen der Zellen eingesetzt werden, um jeweils selektiv bestimmte Zellen bevorzugt zu stimulieren.

Th2-Lymphozyten lassen sich besonders gut kombiniert aktivieren, d.h. unter Verwendung von mehreren Stimulatoren. Hier eignen sich Mitogen und/oder Phorbolester in Kombination mit Antikörpern, zum Beispiel Anti-CD 28. Letzteres ist ein costimulierender Coaktivator. Weiterhin geeignet sind Ionenkanal-bildende Ionenschleuser. Wenn die Stimulatoren konkurrierende Mediatoren, zum Beispiel Interleukin 4 und Interferon gamma aus T-Zellen freisetzen, dann werden vorzugsweise mindestens zwei verschiedene Ansätze gefahren. Dies ist besonders bei der Untersuchung von Blut von Allergikern und Atopikern, wie Neurodermitikern, wichtig, bei denen zuviel Interleukin 4 gebildet wird. Die nicht abschließende Aufzählung zeigt die Vielfalt der durchführbaren Aktivitätsbestimmungen. Es können deshalb den jeweiligen Arten der Bestimmung angepaßte Blutentnahmesysteme, insbesondere Spritzenkolben für die Blutentnahme, die die jeweiligen spezifischen Stimulantien bereits enthalten können, vorrätig gehalten werden.

Gegenstand der Erfindung sind demgemäß auch Testkits zur Bestimmung der Immunabwehraktivität von Blutzellen. Diese Testkits umfassen
a) ein Blutentnahmebesteck mit
b) einem Inkubrationsgefäß, das als zur Blutentnahme geeigneter Spritzenzylinder ausgebildet ist;
c) einen Blutgerinnungsinhibitor, der vorzugsweise in lyophilisierter Form im Blutentnahmesystem, insbesondere im Spritzenzylinder, vorgehalten ist;
d) mindestens ein Stimulans zur Aktivierung der Blutzellen, das vorzugsweise in getrockneter, insbesondere lyophilisierter Form im Blutentnahmesystem, insbesondere im Spritzenzylinder, oder in einem separaten Gefäß vorgehalten ist;
e) eine Kulturlösung für das Kultivieren des entnommenen Blutes; und
f) einen in den Spritzenzylinder passenden Ventilkolben zur Trennung von abgesetzten roten Blutkörperchen vom Überstand im Blutentnahmesystem.

Die Kulturlösung ist vorzugsweise in einer Einstechflasche vorrätig gehalten, wobei das Volumen der Einstechflasche vorzugsweise dem für die Kultivierung geeigneten Volumen an Nährlösungen entspricht. Durch vollständiges Entleeren der Vorratsflasche vor oder nach der Blutentnahme ist dann das richtige Volumen in das Blutentnahmesystem, insbesondere in den Spritzenzylinder, überführt. Der Ventilkolben ist in der Regel außerhalb des Blutentnahmesystems vorgesehen und nach dem Absetzen der Blutkörperchen und nach Beendigung der Inkubation in das Blutentnahmesystem, insbesondere in den Spritzenzylinder, einführbar.

Zur Ausrüstung gehört dann vorzugsweise noch eine oben erwähnte thermostatisch regulierbare Vorrichtung, insbesondere ein Thermoblock, in den die Blutentnahmesysteme, insbesondere die Spritzenzylinder, einsteckbar sind und zwar beispielsweise 20 Spritzenzylinder gleichzeitig, wodurch es möglich ist, mehrere Ansätze gleichzeitig zu kultivieren. Weiterhin gehört hierzu ein geeignetes Kühlgerät und eine ausreichende Anzahl an Kühlblöcken für den Transport. Wenn erwünscht oder besondere Bestimmungen durchgeführt werden sollen, ist es auch möglich, leere oder nur einzelne Substanzen enthaltende Blutentnahmesysteme zu verwenden und in diesen dann die für den Spezialfall gewünschten Substanzen, wie zum Beispiel Kombinationen von Stimulatoren, zuzusetzen.

### Beispiel

In einem Spritzenzylinder, beispielsweise einer Monovette der Firma Sarstedt, ist lyophilisiertes Heparin und opsoniertes Zymosan als mögliches Stimulans vorgelegt. Auf den Spritzenzylinder wird die zugehörige Nadel aufgesetzt, wonach aus einer Einstechflasche der gesamte Inhalt einer Kulturlösung von ca. 3 ml herausgesaugt wird. Blutgerinnungsmittel und Stimulans werden im Nährmedium aufgenommen. Es werden dann der Person, deren Blut untersucht werden soll, 2 ml Blut entnommen, was im Rahmen von Mehrfachuntersuchungen erfolgen kann. Dabei vermischt sich das entnommene Blut mit der Kulturlösung und den darin enthaltenen Substanzen. Nach Entfernen der Spritzennadel bleibt der Spritzenkolben verschlossen, da eine an diesem vorgesehene Schraubenkappe eine selbst abdichtende Durchstechmembran aufweist. Bei nach unten gezogenem Spritzenkolben wird die Kolbenstange durch seitliches Abbiegen an einer Sollbruchstelle abgebrochen, so daß der Spritzenkolben nach Art eines Reagenzglases mit Verschluß verwendbar ist. Der Spritzenkolben wird dann zusammen mit anderen Spritzenkolben, in denen ähnliche Tests durchgeführt werden, in einen Thermoblock gegeben und in diesem bei konstant 37 °C während 24 Stunden inkubiert. Während dieser Zeit werden die Zellen zur Abgabe ihrer Botenstoffe stimuliert, die in das Serum abgegeben werden. Während der Inkubierung setzen sich die Blutkörperchen unten ab, so daß ein im wesentlichen klarer Überstand zurückbleibt. Bei Beendigung der Inkubationszeit werden die Spritzenzylinder aus dem Thermoblock genommen und durch Abnahme der Kappe geöffnet. Mit Hilfe eines Stößels wird ein Ventilkolben, dessen Außendurchmesser dem Innendurchmesser des Spritzenzylinders entspricht, in den Spritzenzylinder eingeführt und langsam durch den Überstand bis kurz über die sedimentierten Blutkörperchen in die Flüssigkeit eingedrückt, wobei sich das Ventil im Ventilkolben öffnet und ein Hindurchfließen des Überstandes in einen über dem Ventilkolben gebildeten separaten Raum veranlaßt. Nach Herausnahme des hierzu verwendeten Stößels, schließt sich das Ventil im Ventilkolben automatisch, und der Spritzenzylinder kann durch Aufsetzen der Schraubenkappe wieder verschlossen werden. Die Blutkörperchen sind auf diese Weise bleibend vom Überstand getrennt, unabhängig davon, welche Erschütterungen der Spritzenkolben danach erfährt. Nach der Trennung von Blutkörperchen und Überstand werden die Spritzenkolben auf eine Temperatur von ca. +4 °C gekühlt und in Kühlblocks gesteckt, die ebenfalls auf diese Temperatur oder auf eine darunterliegende Temperatur gekühlt sind. Die Kühlkapazität dieser Kühlblocks ist so bemessen, daß sie in geeigneten Versandtaschen, zum Beispiel Luftpolstertaschen, während einer Versanddauer von mindestens 24 Stunden, ohne Gefahr eines unerwünschten Temperaturanstieges, ihre Temperatur halten können. Im Testlabor, das sich in der Regel an einem anderen Ort befindet als der Ort der Blutentnahme und der Inkubation, kann dann die Aktivitätsbestimmung, beispielsweise durch ELISA, unter Einsatz vorbestimmter Volumina des Überstandes durchgeführt werden. Parallelversuche haben gezeigt, daß bei diesem Vorgehen genaue und reproduzierbare Aktivitätswerte erhalten werden.

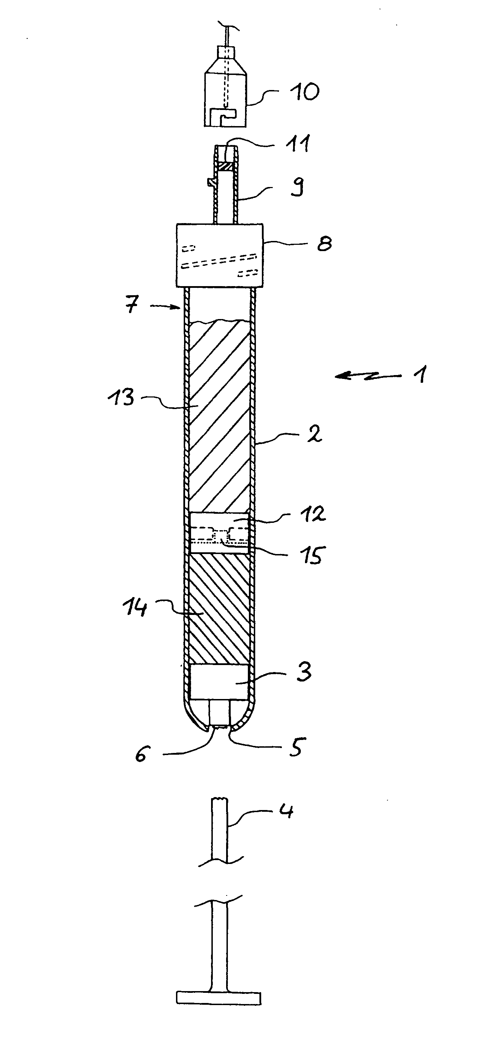

Die beiliegende Zeichnung zeigt eine bevorzugte Ausführungsform eines Spritzenzylinders zur Durchführung des erfindungsgemäßen Verfahrens.

Der Spritzenzylinder 1 weist einen Hohlzylinder 2 auf, in dem ein Spritzenkolben 3 mit Hilfe einer Kolbenstange 4 abdichtend verschiebbar angeordnet ist. Der Hohlzylinder 2 weist im Bereich der Kolbenstange 4 eine Verjüngung 5 auf, die als Führung für die Kolbenstange 4 dient. Die Kolbenstange 4 besitzt in der Nähe des Kolbens 3 eine Sollbruchstelle 6, die bei nach hinten gezogenen Kolben im Bereich der Führung 5 zu liegen kommt und ein müheloses Abbrechen der Kolbenstange bei nach hinten geführtem Kolben ermöglicht. Das von der Kolbenstange 4 abweisende Ende 7 des Hohlzylinders ist über den gesamten Querschnitt offen und mit einer Schraubkappe 8 verschließbar, die einen Aufsteckstutzen 9 für eine Spritzennadel 10 besitzt, die mit einem Bajonettverschluß verriegelbar ist. Der Ansatzstutzen 10 weist im Innern eine durchstechbare Dichtung 11 aus Gummi auf, die beim Aufsetzen der Nadel 10 durchstochen wird und sich beim Abnehmen der Nadel wieder verschließt. Dem Spritzenzylinder 1 ist ein Ventilkolben 12 zugeordnet, der im ungebrauchten Zustand außerhalb des Spritzenzylinders vorgesehen ist und dem ein in der Zeichnung nicht dargestellter Stößel zugeordnet ist. Der Ventilkolben ist mit Hilfe des Stößels bei abgenommener Schraubkappe 8 in den Spritzenzylinder einführbar. Diese Einführung erfolgt nach Beendigung der Inkubation des im Spritzenzylinder enthaltenen Blutes, das sich in einen Überstand 13 und ein Sediment 14 aufgetrennt hat, welches die Blutkörperchen enthält. Zur bleibenden Trennung von Überstand 13 und Sediment 14 wird der Ventilkolben bis zur Grenze zwischen Überstand und Sediment eingedrückt, wobei der Überstand durch das Ventil 15 des Ventilkolbens strömt, bis der Trennvorgang bei Erreichen der Grenze zwischen Überstand und Sediment beendet wird. Bei Beendigung des Eindrückvorganges und Herausnehmen des Stößels schließt das Ventil 15 automatisch. Der Spritzenkolben kann dann wieder durch Aufsetzen der Schraubkappe 8 dicht verschlossen und der Spritzenkolben als Aufbewahrungs-, Kühl- und Transportbehälter verwendet werden, so daß das Blut bzw. seine getrennten Teile zwischen der Blutentnahme und der eigentlichen Durchführung der Aktivitätsbestimmung nicht umgefüllt zu werden braucht. Spritzenzylinder der beschriebenen Art sind unter der Bezeichnung "Monovetten" und Ventilkolben unter der Bezeichnung "Seraplas", jeweils von der Firma Sarstedt, D-51588 Nymbrecht im Handel.

## Patentansprüche

1. Verfahren zur Bestimmung der Immunabwehraktivität von Blut durch Inkubierung des Vollbluts in Gegenwart von mindestens einem Blutgerinnungsinhibitor, wobei das Inkubieren des Blutes im wesentlichen unmittelbar auf die Blutentnahme bei ca. 37 °C erfolgt, und wobei das Blut ohne vorherige Abtrennung der roten Blutkörperchen extrakorporal mit dem mindestens einen Blutgerinnungsinhibitor und mindestens einem Stimulans (Aktivierungsmittel) zur Stimulierung von Blutzellen zur Abgabe von Botenstoffen in Verbindung gebracht wird, das gesamte Blut inkubiert wird, danach eine Trennung des Überstandes von Blutkörperchen im Sediment vorgenommen wird und die Immunabwehraktivität anhand der im Überstand enthaltenen Botenstoffe bestimmt wird, **dadurch gekennzeichnet, dass** die Inkubierung des Vollblutes in Gegenwart von Nährmedium in einem zur Blutentnahme verwendeten Blutentnahmesystem für 8 bis 60 Stunden erfolgt, und die Trennung des Überstandes von Blutkörperchen im Sediment im Blutentnahmesystem ohne vorherige Zentrifugation vorgenommen wird, wobei ein Blutentnahmesystem mit einem beweglichen Spritzenkolben verwendet wird und die Trennung mit Hilfe einer zeitweise durchlässigen Trennwand vorgenommen wird.

2. Verfahren nach Anspruch 1, **dadurch gekennzeichnet, daß** das Inkubieren in Gegenwart von Nährmedium vorgenommen wird, das mit mindestens einem Blutgerinnungsinhibitor und insbesondere auch mindestens einem Stimulans zusammengebracht wird, bevor das Blut zugegeben wird.

3. Verfahren nach Anspruch 1, **dadurch gekennzeichnet, daß** das Inkubieren in Gegenwart von Nährmedium vorgenommen wird, das nach dem Versetzen des Blutes mit mindestens einem Blutgerinnungsinhibitor mit dem Blut in Verbindung gebracht wird.

4. Verfahren nach einem der vorhergehenden Ansprüche, **dadurch gekennzeichnet, daß** das Inkubieren in Gegenwart von mindestens einem Stimulans vorgenommen wird, das nach dem Versetzen des Blutes mit mindestens einem Blutgerinnungsinhibitor mit dem Blut in Verbindung gebracht wird.

5. Verfahren nach einem der vorhergehenden Ansprüche, **dadurch gekennzeichnet, dass** der mindestens eine Blutgerinnungsinhibitor Heparin ist.

6. Verfahren nach einem der vorhergehenden Ansprüche, **dadurch gekennzeichnet, dass** die Inkubierung des Vollblutes in einer thermostatisch regulierbaren Vorrichtung, insbesondere einem Thermoblock, vorgenommen wird.

7. Verfahren nach einem der vorhergehenden Ansprüche, **dadurch gekennzeichnet, daß** das inkubierte Blut nach der Inkubation und Trennung gekühlt wird und bis zur Aktivitätsbestimmung gekühlt gehalten wird.

8. Verfahren nach einem der vorhergehenden Ansprüche, **dadurch gekennzeichnet, dass** das zur Blutentnahme verwendete Blutentnahmesystem ein Spritzenzylinder ist und wobei vorzugsweise die Trennung mit Hilfe einer zeitweise durchlässigen Trennwand mit einem verschiebbaren Ventilkolben vorgenommen wird.

9. Verfahren nach einem der vorhergehenden Ansprüche, **dadurch gekennzeichnet, daß** der mindestens eine Blutgerinnungsinhibitor und vorzugsweise auch das mindestens eine verwendete Stimulans, im Blutentnahmesystem, insbesondere im Spritzenzylinder, oder in einem separaten Gefäß in trockener Form vorgelegt sind.

10. Verfahren nach einem der vorhergehenden Ansprüche, **dadurch gekennzeichnet, daß** das aufgeteilte Blut in gekühltem Zustand, vorzugsweise in einer Kühlpackung, transportiert wird.

11. Verfahren nach einem der vorhergehenden Ansprüche, **dadurch gekennzeichnet, daß** das zur Blutentnahme verwendete Blutentnahmesystem, insbesondere der Spritzenzylinder, nach der Trennung von Überstand und roten Blutkörperchen als Aufbewahrungs-, insbesondere Versandbehälter, verwendet wird.

12. Testkit zur Bestimmung der Immunabwehraktivität von Blutzellen, insbesondere zur Durchführung des Verfahrens nach einem der vorhergehenden Ansprüche, mit
a) einem Blutentnahmebesteck mit
b) einem Inkubationsgefäss,
c) einem Blutgerinnungsinhibitor und
d) mindestens einem Stimulans zur Aktivierung der Blutzellen zur Abgabe von Botenstoffen, **dadurch gekennzeichnet, dass**
e) das Inkubationsgefäss als zur Blutentnahme geeigneter Spritzenzylinder ausgebildet ist und dass
f) ein in den Spritzenzylinder, passender Ventilkolben zur Trennung von abgesetzten Blutkörperchen vom Überstand im Spritzenzylinder sowie
g) eine/ Kulturlösung für das Blut vorgesehen sind.

13. Kit nach Anspruch 12, **dadurch gekennzeichnet, daß** der Blutgerinnungsinhibitor und/oder das mindestens eine Stimulans in vorzugsweise trockener, insbesondere lyophilisierter Form, in dem Spritzenzylinder, enthalten sind.

14. Kit nach Anspruch 12 oder 13, **dadurch gekennzeichnet, dass** die Kulturlösung zusammen mit dem Blutgerinnungsinhibitor und insbesondere mit dem mindestens einen Stimulans im Spritzenzylinder, in dem die Kultivierung durchgeführt wird, vorgelegt ist.

15. Kit nach Anspruch 12 oder 13, **dadurch gekennzeichnet, daß** die Kulturlösung und/oder das mindestens eine Stimulans volumendosiert in mindestens einer Einstechflasche enthalten sind.

16. Kit nach einem der Ansprüche 12 bis 15, **dadurch gekennzeichnet, daß** der Ventilkolben außerhalb des Spritzenzylinders vorgesehen und nach dem Absetzen der Blutkörperchen in den eine abnehmbare Kappe aufweisenden Spritzenzylinder einführbar ist.

## Claims

1. Method of determining the immune response activity of blood by incubating whole blood in the presence of at least one blood coagulation inhibitor, the blood incubation taking place almost immediately after the blood sampling at approximately 37°C, the blood, without prior separation of the red blood cells, being brought into extracorporeal contact with the at least one blood coagulation inhibitor and at least one stimulant (activating agent) for stimulating blood cells to release messenger substances, all of the blood being incubated, a separation of the supernatant from blood cells in the sediment then being carried out, and the immune response activity being determined on the basis of the messenger substances contained in the supernatant, **characterized in that** the incubation of the whole blood takes place for 8 to 60 hours in the presence of nutrient medium in a blood sampling system used for sampling the blood, and the separation of the supernatant from blood cells in the sediment is carried out in the blood sampling system without prior centrifuging, a blood sampling system with a movable syringe plunger being used, and the separation being carried out with the aid of a dividing wall which is temporarily permeable.

2. Method according to Claim 1, **characterized in that** the incubation is carried out in the presence of nutrient medium which is brought together with at least one blood coagulation inhibitor and in particular also with at least one stimulant before the blood is added.

3. Method according to Claim 1, **characterized in that** the incubation is carried out in the presence of nutrient medium which is brought into contact with the blood after at least one blood coagulation inhibitor has been added to the blood.

4. Method according to one of the preceding claims, **characterized in that** the incubation is carried out in the presence of at least one stimulant which is brought into contact with the blood after at least one blood coagulation inhibitor has been added to the blood.

5. Method according to one of the preceding claims, **characterized in that** the at least one blood coagulation inhibitor is heparin.

6. Method according to one of the preceding claims, **characterized in that** the incubation of the whole blood is carried out in a thermostatically controllable device, in particular in a thermoblock.

7. Method according to one of the preceding claims, **characterized in that** the incubated blood is cooled after the incubation and separation and is kept cooled up to the time when the activity is determined.

8. Method according to one of the preceding claims, **characterized in that** the blood sampling system used for the blood sampling is a syringe barrel, and the separation is preferably carried out with the aid of a temporarily permeable dividing wall with a displaceable valve plunger.

9. Method according to one of the preceding claims, **characterized in that** the at least one blood coagulation inhibitor, and preferably also the at least one stimulant used, is introduced beforehand in dry form into the blood sampling system, in particular into the syringe barrel, or into a separate vessel.

10. Method according to one of the preceding claims, **characterized in that** the separated blood is transported in a cooled state, preferably in a cooling pack.

11. Method according to one of the preceding claims, **characterized in that**, after separation of supernatant and red blood cells, the blood sampling system used for the blood sampling, in particular the syringe barrel, is used as a storage container, in particular as a dispatch container.

12. Test kit for determining the immune response activity of blood cells, in particular for carrying out the method according to one of the preceding claims, with
a) blood sampling equipment with
b) an incubation vessel,
c) a blood coagulation inhibitor, and
d) at least one stimulant for activating the blood cells to release messenger substances,
**characterized in that**
e) the incubation vessel is designed as a syringe barrel suitable for blood sampling, and **in that**
f) a valve plunger which fits into the syringe barrel and is used to separate settled blood cells from the supernatant in the syringe barrel, and
g) a culture solution for the blood are provided.

13. Kit according to Claim 12, **characterized in that** the blood coagulation inhibitor and/or the at least one stimulant are contained in the syringe barrel in a preferably dry form, in particular a freeze-dried form.

14. Kit according to Claim 12 or 13, **characterized in that** the culture solution is placed together with the blood coagulation inhibitor and in particular with the at least one stimulant in the syringe barrel in which the culturing is performed.

15. Kit according to Claim 12 or 13, **characterized in that** the culture solution and/or the at least one stimulant are contained in dosed volumes in at least one pierceable vial.

16. Kit according to one of Claims 12 to 15,
**characterized in that** the valve plunger is provided outside the syringe barrel and, after settling of the blood cells, it can be introduced into the syringe barrel, the latter having a removable cap.

## Revendications

1. Procédé de détermination de l'activité de la réponse immunitaire du sang par incubation du sang complet en présence d'au moins un inhibiteur de coagulation du sang, l'incubation du sang s'effectuant essentiellement tout de suite après le prélèvement du sang et à environ 37°C, le sang étant amené sans séparation préalable des globules rouges du sang et extracorporellement en contact avec l'au moins un inhibiteur de la coagulation du sang et au moins un stimulant (moyen d'activation) en vue de stimuler les cellules sanguines pour qu'elles délivrent des substances messagères, le sang complet étant incubé, une séparation du surnageant des corpuscules du sang présents dans le sédiment étant réalisée et l'activité de réponse immunitaire étant déterminée à l'aide des substances messagères contenues dans le surnageant, **caractérisé en ce que** l'incubation du sang complet s'effectue pendant 8 à 60 heures en présence d'un milieu nutritif dans le système de prélèvement du sang utilisé pour le prélèvement du sang, la séparation du surnageant et des corpuscules du sang présents dans le sédiment du système de prélèvement du sang étant réalisée sans centrifugation préalable, le système utilisé pour le prélèvement du sang étant doté d'un piston d'injection mobile et la séparation étant réalisée à l'aide d'une paroi de séparation dont la perméabilité varie dans le temps.

2. Procédé selon la revendication 1, **caractérisé en ce que** l'incubation est réalisée en présence d'un milieu nutritif auquel au moins un inhibiteur de coagulation du sang et en particulier au moins un stimulant sont ajoutés avant que le sang soit ajouté.

3. Procédé selon la revendication 1, **caractérisé en ce que** l'incubation est réalisée en présence d'un milieu nutritif qui est mis en contact avec le sang après le mélange du sang avec au moins un inhibiteur de la coagulation du sang.

4. Procédé selon l'une des revendications précédentes, **caractérisé en ce que** l'incubation est réalisée en présence d'au moins un stimulant qui est mis en contact avec le sang après le mélange du sang avec au moins un inhibiteur de la coagulation du sang.

5. Procédé selon l'une des revendications précédentes, **caractérisé en ce que** l'au moins un inhibiteur de coagulation du sang est l'héparine.

6. Procédé selon l'une des revendications précédentes, **caractérisé en ce que** l'incubation du sang complet est réalisée dans un dispositif thermostatisé et en particulier dans un thermobloc.

7. Procédé selon l'une des revendications précédentes, **caractérisé en ce que** le sang incubé est refroidi après l'incubation et la séparation et est maintenu froid jusqu'à la détermination de l'activité.

8. Procédé selon l'une des revendications précédentes, **caractérisé en ce que** le système de prélèvement de sang utilisé pour le prélèvement du sang est un cylindre de seringue et dans lequel la séparation set réalisée de préférence à l'aide d'une paroi de séparation dont la perméabilité varie dans le temps et d'un piston mobile à soupape.

9. Procédé selon l'une des revendications précédentes, **caractérisé en ce que** l'au moins un inhibiteur de coagulation du sang et de préférence aussi l'au moins un stimulant utilisés sont placés sous forme séchée dans le système de prélèvement du sang et en particulier dans le cylindre de la seringue ou dans un récipient séparé.

10. Procédé selon l'une des revendications précédentes, **caractérisé en ce que** le sang divisé est transporté à froid, de préférence dans un emballage refroidi.

11. Procédé selon l'une des revendications précédentes, **caractérisé en ce qu'**après la séparation du surnageant et des corpuscules rouges du sang, le système de prélèvement du sang utilisé pour le prélèvement du sang et en particulier le cylindre de seringue sont utilisés comme récipient de conservation et en particulier d'expédition.

12. Trousse de test en vue de la détermination de l'activité de réponse immunologique de cellules du sang, en particulier en vue de la mise en oeuvre du procédé selon l'une des revendications précédentes, qui comporte :
a) une trousse de prélèvement de sang qui comprend
b) un récipient d'incubation,
c) un inhibiteur de coagulation du sang et
d) au moins un stimulant en vue de l'activation des cellules du sang pour qu'elles délivrent des substances messagères,
**caractérisée en ce que**
e) le récipient d'incubation est configuré comme cylindre de seringue qui convient pour le prélèvement du sang et **en ce que**
f) un piston à soupape adapté au cylindre de seringue est prévu pour la séparation des corpuscules du sang qui se sont déposés et du surnageant dans le cylindre de seringue, ainsi que
g) une solution de culture du sang.

13. Trousse selon la revendication 12, **caractérisée en ce que** l'inhibiteur de coagulation du sang et/ou l'au moins un stimulant sont contenus dans le cylindre de seringue de préférence sous forme sèche et en particulier sous forme lyophilisée.

14. Trousse selon les revendications 12 ou 13,
**caractérisée en ce que** la solution de culture est placée en même temps que l'inhibiteur de coagulation du sang et en particulier que l'au moins un stimulant dans le cylindre de seringue dans lequel la culture est réalisée.

15. Trousse selon l'une des revendications 12 ou 13, **caractérisée en ce qu'**un volume dosé de solution de culture et/ou de l'au moins un stimulant est placé dans au moins un flacon perforable.

16. Trousse selon l'une des revendications 12 à 15, **caractérisée en ce que** le piston de soupape est prévu à l'extérieur du cylindre de seringue et peut être inséré dans le cylindre de seringue qui présente un bonnet amovible après la sédimentation des corpuscules du sang.
